# DEMANDE DE BREVET EUROPEEN

(11) **EP 2 140 778 A1**
(43) Date de publication de la demande: **06.01.2010**
(21) Numéro de dépôt: 09290532.2
(22) Date de dépôt: 02.07.2009
(51) Int. Cl.: A46B 9/02, A61M 35/00, B65D 83/02, A45D 40/24, A45D 29/04, A45D 29/16, A45D 40/26

(54) **Dispositif de distribution d'accessoires cosmétiques et procédé de préparation associé**

(30) Priorité: 02.07.2008 FR 0854507
(71) Demandeur: L'Oreal, 75008 Paris (FR)
(72) Inventeur: Thiebaut, Laure, 92110 Clichy (FR)
(74) Mandataire: Neyret, Daniel Jean Marie

(57) **Abrégé**

Ce dispositif (10) comprend un support (12) d'accessoires comprenant des accessoires cosmétiques (34) détachables. Il comprend un distributeur (14) d'accessoires cosmétiques délimitant un chemin de guidage (30) du support (12) d'accessoires.

Le support (12) d'accessoires comprend au moins un chapelet (32) d'accessoires cosmétiques (34) disposés bout à bout en étant reliés entre eux par des liaisons frangibles (36). La rupture d'une des liaisons frangibles (36) pour détacher un accessoire cosmétique (34) du support (32) provoque une diminution de la longueur du chapelet (32) d'accessoires, prise le long du chemin de guidage (30).

## Description

La présente invention concerne un dispositif de distribution d'accessoires cosmétiques, du type comprenant :
- au moins un support d'accessoires comprenant une pluralité d'accessoires cosmétiques, chaque accessoire cosmétique étant détachable par rapport aux autres accessoires cosmétiques du support d'accessoires ;
- un distributeur d'accessoires cosmétiques délimitant un chemin de guidage du support d'accessoires, le support d'accessoires étant déplaçable suivant sa longueur dans le chemin de guidage entre une position rétractée dans le distributeur et une pluralité de positions déployées d'utilisation d'un accessoire.

Ces accessoires sont notamment des applicateurs de produit cosmétique présentant une partie active destinée à entrer en contact avec la peau ou les fibres kératiniques d'un utilisateur, ou encore des outils de traitement cosmétique tels que des limes à ongles ou des roulettes de massage.

Par « produit cosmétique », on entend notamment un produit tel que défini dans la directive 93/35/CEE du Conseil du 14 juin 1993.

Un tel dispositif, lorsqu'il comprend un applicateur de produit cosmétique, est destiné par exemple à déposer un produit cosmétique sur des fibres kératiniques, telles que des cheveux ou des cils, y compris des extensions capillaires ou des extensions de cils.

Le produit est avantageusement un produit cosmétique de maquillage et/ou de soin cosmétique comme par exemple un mascara.

Par « mascara » on entend une composition destinée à être appliquée sur les cils. Il peut s'agir d'une composition de maquillage des cils, d'une composition de traitement cosmétique des cils, d'une base de maquillage des cils, ou encore d'une composition dite « top-coat » appliquée sur une première couche, elle-même déjà appliquée sur les cils.

Pour appliquer des formules classiques de mascara, il est connu d'utiliser un conditionnement comprenant un applicateur et un réservoir de produit cosmétique.

Toutefois, certaines formules de mascara, du fait de leur nature chimique ou physique, ne peuvent pas être retirées de l'applicateur après l'application du mascara sur les cils.

Ainsi, certaines formules cosmétiques subissent une modification chimique et/ou physique lorsqu'elles sont extraites du réservoir, par exemple lorsqu'elles contiennent un produit réactif avec l'oxygène. Dans d'autres cas, la formule sèche rapidement sur l'applicateur et il est difficile de retirer le résidu de formule séchée accroché sur l'applicateur.

L'applicateur peut alors devenir inutilisable et ne plus permettre une séparation efficace des cils.

En outre, lorsqu'un applicateur souillé est replongé dans le réservoir de produit cosmétique, il peut contaminer le produit cosmétique contenu dans le réservoir. Cette contamination peut entraîner, notamment dans les formules dépourvues de conservateur, un assèchement de la formule, ou encore un changement de couleur, de viscosité ou d'aspect.

Dans tous les cas énoncés précédemment, il est nécessaire de prévoir des applicateurs à usage unique qui peuvent être remplacés après chaque application de produit.

Par ailleurs, un tel remplacement après chaque usage peut s'avérer nécessaire, lorsque le même réservoir de produit cosmétique est utilisé pour appliquer du produit cosmétique sur plusieurs utilisatrices, par exemple au sein d'un institut de beauté.

Pour le cas où l'applicateur doit être remplacé après chaque utilisation, on connaît des dispositifs de distribution comprenant une pluralité d'applicateurs à usage unique.

Un tel dispositif de distribution est par exemple décrit dans le document US 3 690 452. Ce dispositif comprend un étui de distribution et un support continu d'applicateurs pouvant être extrait longitudinalement de l'étui. Le support continu comprend une membrure longitudinale, sur laquelle sont fixés transversalement de manière détachable une pluralité d'applicateurs à usage unique.

Avant chaque utilisation, l'utilisateur ouvre l'étui, puis déplace la membrure vers l'extérieur et détache un applicateur en vue de l'application de produit.

Un tel dispositif ne donne pas entière satisfaction. En effet, il est très encombrant, puisque chaque applicateur comprend un corps suffisamment long pour permettre la préhension par les doigts de l'utilisateur et l'application de produit. Du fait de sa taille, ce dispositif est coûteux à réaliser.

Un but de l'invention est d'obtenir un dispositif de distribution d'accessoires cosmétiques comportant une pluralité d'accessoires à usage unique, et qui soit peu encombrant et peu coûteux.

A cet effet, l'invention a pour objet un dispositif du type précité, **caractérisé en ce que** le support d'accessoires comprend au moins un chapelet d'accessoires cosmétiques disposés bout à bout en étant reliés entre eux par des liaisons frangibles, la rupture d'une des liaisons frangibles pour détacher un accessoire cosmétique du support provoquant une diminution de la longueur du chapelet d'accessoires, prise le long du chemin de guidage.

Le dispositif comprend l'une ou plusieurs des caractéristiques suivantes, prise(s) isolément ou suivant toute(s) combinaison(s) techniquement possible(s) :
- la pluralité d'accessoires cosmétiques et les liaisons frangibles du chapelet d'accessoires sont venues de matière ;
- chaque accessoire cosmétique comprend :

- une base longitudinale raccordée à une base longitudinale d'au moins un accessoire adjacent par une liaison frangible ; et
- au moins une partie transversale qui fait saillie transversalement par rapport à la base longitudinale ;
- la base et la partie transversale forment un embout applicateur de produit cosmétique ;
- la partie transversale comprend un bras de liaison qui fait saillie transversalement par rapport à la base longitudinale, et un embout applicateur de produit cosmétique disposé à une extrémité libre du bras ;
- chaque liaison frangible comprend un organe longitudinal présentant un premier point de liaison avec un premier accessoire du chapelet d'accessoires et un deuxième point de liaison avec un deuxième accessoire du chapelet d'accessoires ;
- au moins le premier accessoire présente un bord de protection d'utilisateur qui fait saillie longitudinalement par rapport au premier point de liaison vers le deuxième accessoire, le bord de protection s'étendant longitudinalement en regard de l'organe longitudinal ;
- le chemin de guidage défini par le distributeur est linéaire, le support d'accessoires s'étendant linéairement au moins lorsqu'il est disposé dans le chemin de guidage du distributeur ;
- le chemin de guidage défini par le distributeur est curviligne, le support d'accessoires adoptant une configuration curviligne au moins lorsqu'il est disposé dans le chemin de guidage du distributeur ;
- le support d'accessoires comprend un organe de déplacement du chapelet d'accessoires dans le distributeur, monté solidaire et distinct du chapelet d'accessoires, l'organe de déplacement étant propre à être saisi par un utilisateur du dispositif à travers le distributeur pour déplacer le chapelet d'accessoires dans le distributeur le long du chemin de guidage ;
- le distributeur comprend :

- un corps creux de réception du support d'accessoires ; et
- une tête d'appui faisant saillie à partir du corps creux, le chemin de guidage s'étendant à travers le corps creux et à travers la tête d'appui, chaque accessoire étant recouvert par le corps creux pour empêcher l'utilisation de l'accessoire lorsqu'il est disposé dans le corps creux, l'espace situé au-dessus d'un accessoire étant dégagé pour autoriser l'utilisation de l'accessoire, lorsqu'il est disposé dans la tête d'appui ;
- le chemin de guidage du distributeur est délimité par une rainure ou une gorge de section transversale sensiblement conjuguée à la section transversale d'au moins un accessoire cosmétique du support d'accessoires ; et
- chaque accessoire cosmétique est choisi parmi un applicateur de produit cosmétique ou un outil de traitement cosmétique.

L'invention a également pour objet un procédé de préparation d'un dispositif tel que défini ci-dessus en vue de l'utilisation d'un accessoire cosmétique, **caractérisé en ce qu**'il comprend les étapes suivantes :
- déplacement du support d'accessoires dans le chemin de guidage jusqu'à une position déployée ;
- rupture d'une liaison frangible entre un premier accessoire cosmétique et un deuxième accessoire cosmétique, la rupture de la liaison frangible et le détachement du premier accessoire cosmétique provoquant une diminution de la longueur du chapelet d'accessoires, prise le long du chemin de guidage.

L'invention sera mieux comprise à la lecture de la description qui va suivre, donnée uniquement à titre d'exemple, et faite en se référant aux dessins annexés, sur lesquels :
- la Figure 1 est une vue en perspective de dessous d'un premier dispositif de distribution selon l'invention, un support continu d'accessoires étant disposé dans le distributeur ;
- la Figure 2 est une vue analogue à la Figure 1 du support continu d'accessoires ;
- la Figure 3 est une vue d'un détail marqué III de la Figure 2 ;
- la Figure 4 est une vue partielle de côté de l'extrémité du dispositif de la Figure 1, lors de différentes étapes du procédé d'utilisation de ce dispositif ;
- la Figure 5 est une vue analogue à la Figure 4, illustrant la diminution progressive de longueur du support continu d'accessoires du dispositif de la Figure 1 ;
- la Figure 6 est une vue partielle de côté du support d'accessoires d'un deuxième dispositif d'application selon l'invention ;
- La Figure 7 est une vue de dessus, avec écorchement partiel, d'un troisième dispositif de distribution selon l'invention ;
- La Figure 8 est une vue illustrant différents types d'accessoires cosmétiques pour des dispositifs selon l'invention ;
- la Figure 9 est une vue analogue à la Figure 1 d'un quatrième dispositif de distribution selon l'invention ;
- la Figure 10 est une vue analogue à la Figure 2 du support continu d'accessoires du quatrième dispositif selon l'invention ;
- la Figure 11 est une vue en perspective partiellement coupée suivant un plan vertical médian d'un cinquième dispositif de distribution selon l'invention ;
- la Figure 12 est une vue de côté du support continu d'accessoires du cinquième dispositif selon l'invention ;
- la Figure 13 est une vue en perspective de dessus d'un sixième dispositif de distribution selon l'invention ;
- la Figure 14 est une vue de côté du support continu d'accessoires du sixième dispositif selon l'invention ;
- la Figure 15 est une vue analogue à la Figure 13 d'un septième dispositif de distribution selon l'invention ;
- la Figure 16 est une vue en perspective de dessus du support continu d'accessoires du dispositif de la Figure 15 avant chargement du produit cosmétique ;
- la Figure 17 est une vue analogue à la Figure 16 du support d'accessoires d'un huitième dispositif selon l'invention après chargement du produit cosmétique.

Dans tout ce qui suit, les orientations sont données relativement aux figures. Les termes « avant » et « arrière » s'entendent de manière relative par rapport à la position d'un utilisateur lors de l'utilisation du dispositif, le terme « avant » s'entendant comme plus près du point d'utilisation de l'accessoire sur l'utilisateur, alors que le terme « arrière » s'entend comme plus éloigné de ce point.

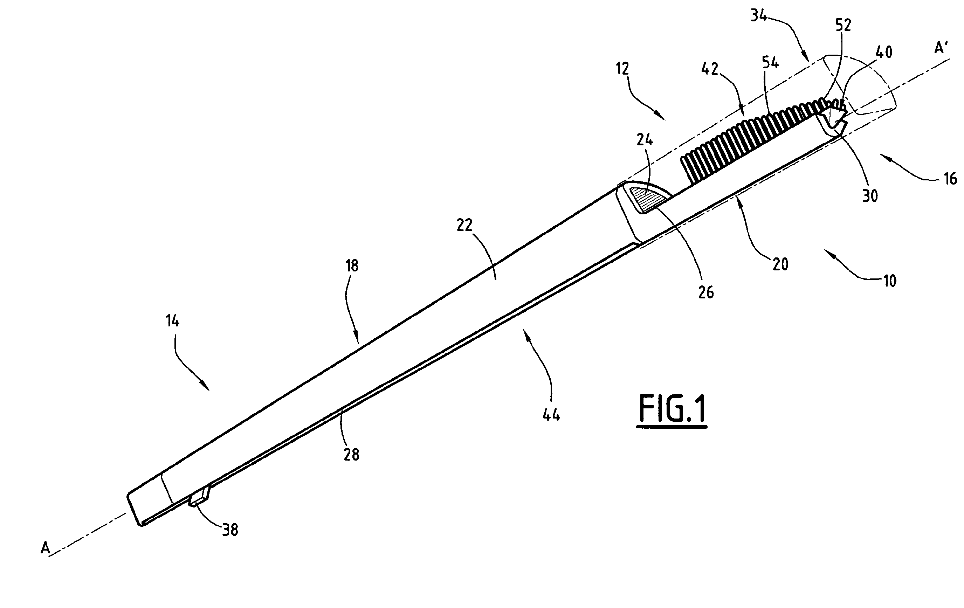

Les Figures 1 à 5 illustrent un premier dispositif 10 de distribution d'accessoires cosmétiques selon l'invention. Dans cet exemple, le dispositif 10 est destiné à l'application d'un produit cosmétique sur les fibres kératiniques d'un utilisateur, à l'aide d'un accessoire cosmétique à usage unique formé par un applicateur de produit cosmétique.

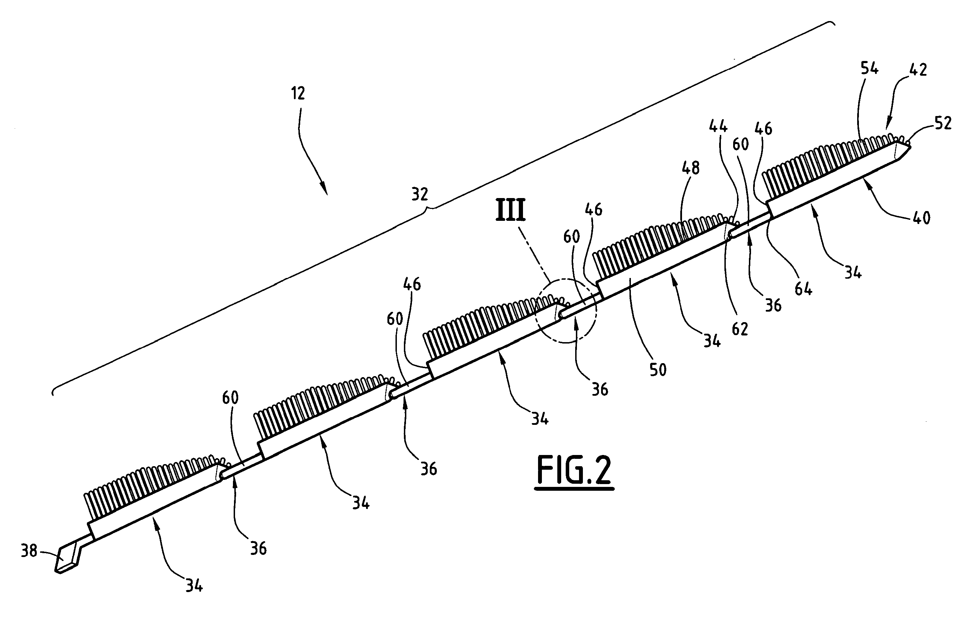

Ce dispositif 10 comprend un support continu 12 d'accessoires cosmétiques détachables, visible notamment sur la Figure 2, et un distributeur 14 d'accessoires, propre à distribuer et à porter successivement les différents accessoires cosmétiques du support continu 12, lorsque le support 12 est introduit dans le distributeur 14.

Dans cet exemple, le dispositif 10 comprend en outre, tel qu'illustré schématiquement sur la figure 1, un réservoir 16 de produit cosmétique formant un capuchon d'obturation du distributeur 14.

Le distributeur 14 s'étend suivant un axe longitudinal A-A'. Il comprend un organe de préhension destiné à être saisi par l'utilisateur, l'organe de préhension étant formé dans cet exemple par un corps tubulaire creux 18 d'axe A-A'. Le dispositif 10 comprend en outre une tête 20 d'appui d'accessoires qui fait saillie longitudinalement à une extrémité avant du corps 18.

Le corps 18 comprend une paroi périphérique tubulaire 22 sensiblement pleine, d'axe A-A'. La paroi 22 définit intérieurement une lumière de circulation 24 du support continu 12 d'accessoires.

La lumière 24 débouche axialement à une extrémité avant du corps 18, en regard de la tête d'appui 20, et débouche transversalement vers le bas par une fente de guidage 28.

La fente de guidage 28 s'étend longitudinalement le long d'une génératrice de la paroi 22.

La paroi périphérique 22 présente, le long de la fente 18, des saillies ou grains de riz (non représentés) d'indexation du déplacement du support continu 12, qui font saillie dans la fente 28, pour former des butées libérables d'immobilisation du support 12.

La paroi 22 recouvre les accessoires du support continu 12 qui sont disposés dans la lumière 24, de sorte que les accessoires du support 12 ne peuvent pas être utilisés lorsqu'ils sont disposés dans la lumière 24.

La tête d'appui 20 s'étend longitudinalement vers l'avant dans le prolongement du corps 18 et de la fente 28.

Dans un premier mode de réalisation, la tête d'appui 20 est monobloc avec le corps tubulaire creux 18. En variante, la tête 20 est rapportée sur le corps tubulaire creux 18 en étant fixée par tout moyen de fixation approprié tel que par ajustement serré, par encliquetage, par vissage, par collage ou par sertissage.

La tête 20 est ouverte vers le haut. Ainsi, lorsqu'un accessoire du support 12 est disposé dans la tête 20, l'espace disposé au-dessus de l'accessoire est dégagé et l'accessoire peut être utilisé.

La paroi périphérique 22 située de part et d'autre de la fente 28, et la tête d'appui 20 définissent un chemin de guidage profilé 30 en forme de rainure pour le support continu d'accessoires 12. Le chemin de guidage 30 s'étend linéairement parallèlement à l'axe A-A' et présente une section transversale sensiblement en forme de V.

Dans l'exemple représenté sur la Figure 1, le réservoir 16 de produit cosmétique est fixé de manière libérable sur l'organe de préhension 18 pour couvrir la tête 20. Le réservoir 16 et l'organe tubulaire 18 sont alors munis de moyens de fixation libérables et réversibles tel qu'un filetage disposé par exemple sur le réservoir 16 et engagé sur un pas de vis solidaire de l'organe 18.

En variante, le réservoir 16 est indépendant du distributeur 14 et peut être commun à plusieurs distributeurs 14.

Comme illustré par la Figure 2, le support continu 12 d'accessoires comprend un chapelet 32 d'accessoires cosmétiques 34 reliés entre eux par des liaisons frangibles 36. Le support 12 comprend en outre un bouton 38 de commande du déplacement du chapelet 32 dans le distributeur 14, le long du chemin de guidage 30.

Par « chapelet », on entend, au sens de la présente invention, une succession d'éléments identiques ou analogues reliés entre eux par des liaisons mécaniques.

Par « support continu », on entend que tous les accessoires 34 solidaires du support 12 et les liaisons frangibles 36 reliant entre eux les accessoires 34 sont déplaçables conjointement dans au moins deux sens suivant la longueur du support 12.

Dans l'exemple représenté sur la Figure 2, le bouton 38, les accessoires 34, et les liaisons frangibles 36 sont venus de matière et réalisés d'un seul tenant par moulage par injection d'une matière plastique ou métallique telle que du polypropylène (PP), du polyéthylène (PE), du polyamide (PA), du polyester, du polyéthylène téréphtalate (PET), du polychlorure de vinyle (PVC), du polymétacrylate de méthyle (POMM), ou du ZAMAK.

En variante, des accessoires 34 et les liaisons frangibles 36 sont réalisés d'un seul tenant par bi-injection d'une matière rigide destinée à former les liaisons frangibles 36 et d'une matière souple destinée à former des accessoires 34.

Le coût du support continu 12 est donc très faible.

Les accessoires 34 du chapelet 32 sont, dans cet exemple, des applicateurs de produit cosmétique. Ils sont tous identiques et sont disposés bout à bout le long de l'axe A-A' en étant reliés entre eux par des liaisons frangibles 36.

Ainsi, chaque accessoire 34 est relié à chaque extrémité longitudinale à au plus un accessoire 34 adjacent par une liaison frangible 36.

Le chapelet 32 d'accessoires 34 du support 12 comprend N accessoires disposés bout à bout raccordés entre eux par N-1 liaison frangibles. Le nombre d'accessoires N est initialement supérieur à 2 et est avantageusement compris entre 2 et 12.

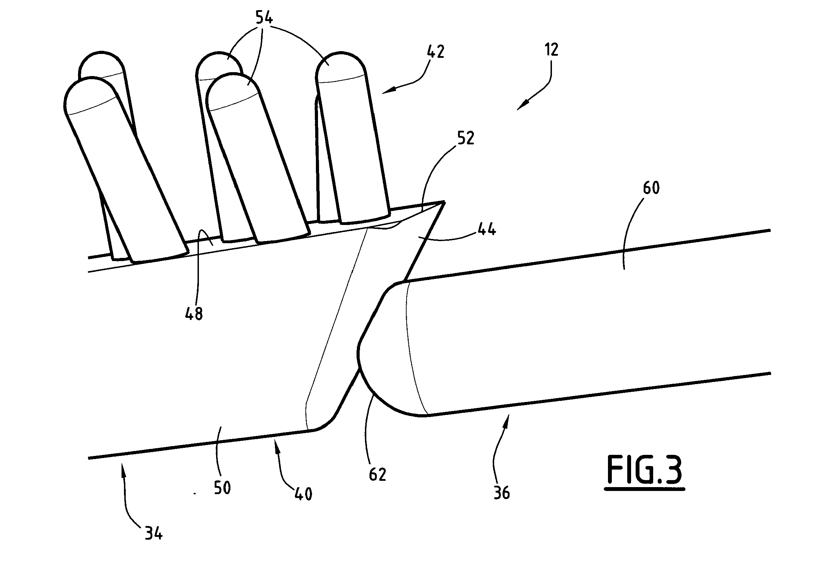

Comme illustré par les Figures 2 et 3, chaque accessoire 34 comprend une base 40 s'étendant sensiblement longitudinalement et au moins un élément transversal 42 d'application de produit cosmétique qui fait saillie transversalement par rapport à la base 40.

Dans l'exemple représenté par la Figure 2, la base 40 présente une section longitudinale passant par l'axe A-A' sensiblement en forme de trapèze et une section transversale triangulaire, sensiblement conjuguée à la section transversale du chemin de guidage 30.

La base 40 s'étend ainsi longitudinalement entre une paroi avant 44 et une paroi arrière 46. Elle présente une paroi supérieure 48 sensiblement plane et deux parois latérales 50 en V raccordant les parois 44, 46.

La paroi avant 44 présente une section longitudinale, prise dans un plan vertical passant par l'axe A-A', inclinée par rapport à un plan perpendiculaire à l'axe A-A'. Elle délimite ainsi un bord avant 52 de protection qui fait saillie vers l'avant, vers un accessoire 34 adjacent. Le bord avant 52 s'étend longitudinalement en regard de la liaison frangible 36.

La paroi supérieure 48 s'étend sensiblement dans un plan horizontal. Elle obture vers le haut les parois latérales 50 et les parois avant 44 et arrière 46.

Les parois latérales 50 sont disposées en appui contre la paroi périphérique 22 du distributeur 14 lorsque l'accessoire 34 est disposé dans le corps 18, et en appui contre la tête d'appui 20 lorsque l'accessoire 34 est disposé dans la tête 20.

Dans l'exemple représenté sur les Figures 1 à 5, les éléments d'application transversaux 42 sont formés par des dents 54 de séparation/peignage qui font saillie vers le haut à l'écart de la paroi supérieure 48.

Dans l'exemple représenté sur la Figure 2, les éléments transversaux 42 comprennent trois rangées de dents 54 longitudinales de hauteur croissante, puis constante en se déplaçant axialement depuis l'avant vers l'arrière de l'accessoire 34. Chaque rangée forme un peigne d'application de produit cosmétique.

Dans cet exemple, la base 40 et les éléments d'application 42 délimitent un embout d'application de produit cosmétique destiné à entrer en contact avec le produit cosmétique et les fibres kératiniques de l'utilisateur. L'embout d'application s'étend longitudinalement parallèlement à l'axe A-A'.

Comme illustré par la Figure 3, chaque liaison frangible 36 comprend une tige de liaison 60 s'étendant longitudinalement suivant l'axe A-A' entre deux accessoires 34 adjacents.

La tige 60 est fixée de manière frangible à son extrémité arrière en un point de liaison 62 frangible sur la paroi avant 44 d'un premier accessoire 34. La tige 60 est fixée de manière solide à son extrémité avant par un point de liaison 64 sur la paroi arrière 46 d'un deuxième accessoire 34 en regard.

La tige 60 présente une section transversale inférieure à la section transversale de la base 40 de l'accessoire 34, par exemple au moins deux fois inférieure à cette section. La section transversale de la tige 60 au niveau du point de liaison frangible 62 est en outre inférieure à la section moyenne de la tige 60.

Le bord avant 52 de la paroi 44 fait saillie longitudinalement à l'avant du point de liaison 62, ce qui limite le risque de blessure de l'utilisateur lors de l'utilisation de l'accessoire après que la liaison frangible 36 a été rompue au niveau du point de liaison frangible 62.

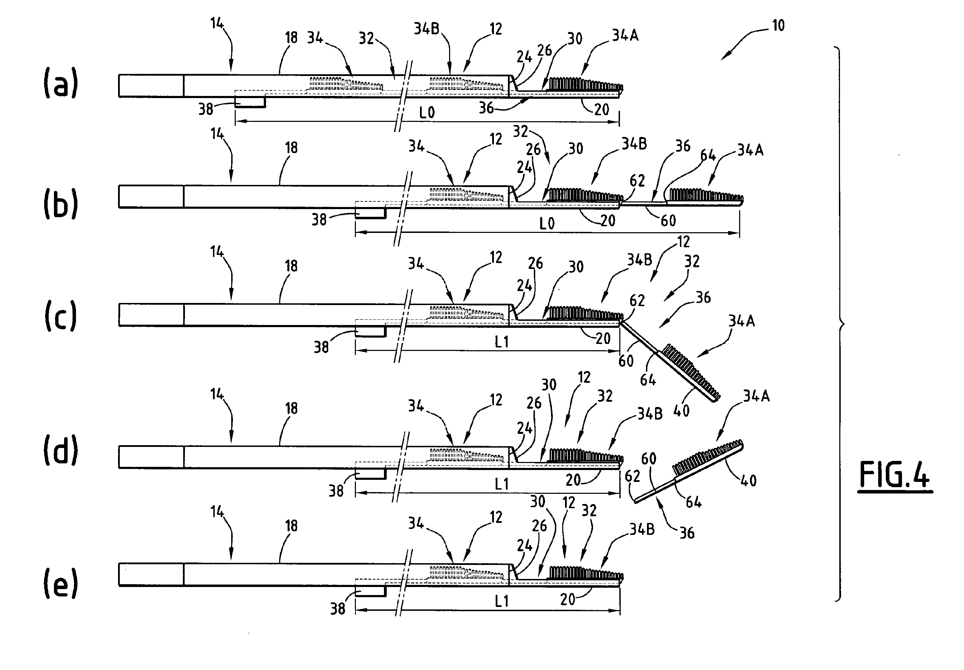

Comme on le verra plus bas en regard de la Figure 4, chaque accessoire 34A est détachable du support continu 12 par rupture d'au moins une liaison frangible 36 au niveau du point de liaison 62. Après rupture de la liaison frangible 36, l'accessoire 34A reste solidaire d'une tige 60 au niveau du point de liaison 64.

Selon l'invention et comme illustré par la Figure 4, les accessoires 34A, 34B étant montés bout à bout en étant raccordés par les liaisons frangibles 36, la longueur L1 du support continu 12, et la longueur correspondante du chapelet 32, prises longitudinalement le long du chemin de guidage 30, après rupture de la liaison frangible 36 et détachement d'un accessoire 34A sont inférieures respectivement à la longueur L0 du support continu 12, et à la longueur correspondante du chapelet 32, prises longitudinalement le long du chemin de guidage 30, avant rupture de la liaison frangible 36, lorsque l'accessoire 34A est solidaire du support continu 12.

Le bouton de commande 38 est distinct des accessoires 34 et des liaisons frangibles 36. Il est fixé longitudinalement à l'arrière de l'accessoire 34 situé à l'extrémité arrière du chapelet 32.

Lorsque le support continu d'accessoires 12 est disposé dans le chemin de guidage 30 du distributeur 14, le bouton 38 fait saillie partiellement hors du distributeur à travers la fente 28, extérieurement par rapport à la paroi périphérique 22, pour être accessible par un doigt de l'utilisateur.

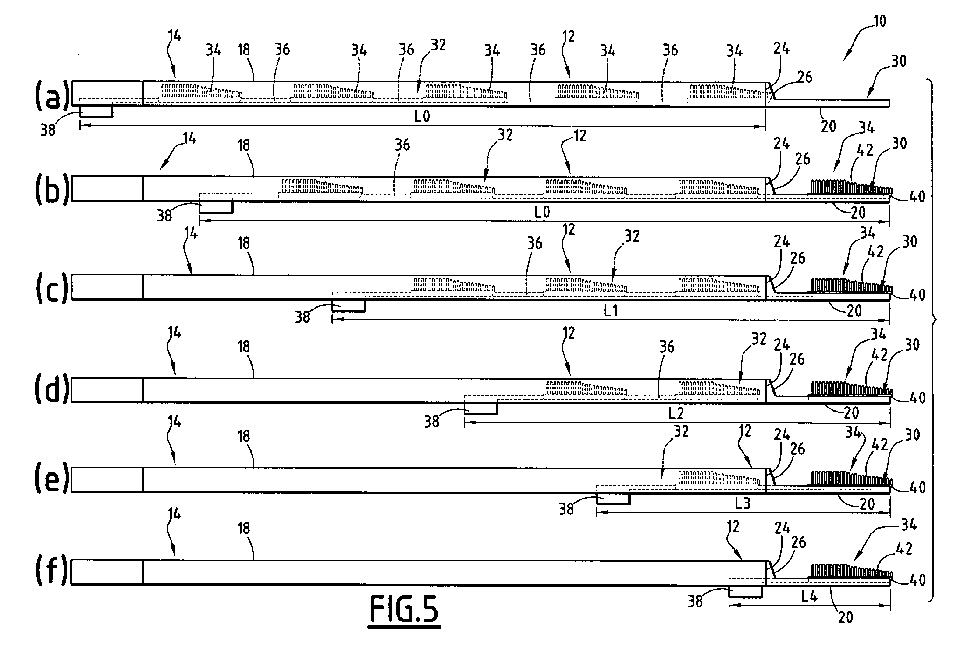

Le bouton de commande 38, chaque accessoire 34, et chaque liaison frangible 36 sont déplaçables conjointement par rapport au distributeur 14 le long du chemin de guidage 30 entre une position totalement rétractée représentée en haut de la Figure 5 et une pluralité de positions déployées représentées de haut en bas sur la Figure 5.

Le fonctionnement du premier dispositif 10 selon l'invention va maintenant être décrit.

Initialement, le dispositif 10 est fourni avec un distributeur 14 et au moins un support continu d'accessoires 12 contenant une pluralité d'accessoires cosmétiques 34, reliés bout à bout par des liaisons frangibles 36.

Le support continu d'accessoires 12 est placé dans le chemin de guidage 30, dans une position totalement rétractée, avec tous les accessoires 34 situés dans la lumière 24 en étant inaccessibles pour l'utilisateur. Il présente une longueur initiale maximale L0, sensiblement égale à la longueur du corps 18, comme illustré en haut de la Figure 5.

Le bouton de commande 38 est alors situé en butée contre l'extrémité arrière de la fente 28. La tête d'appui 20 ne contient aucun accessoire 34.

Lorsque l'utilisateur souhaite utiliser un accessoire cosmétique 34, il saisit le corps 18 avec sa main et déplace le bouton de commande 38 vers l'avant et la tête d'appui 20.

Lors de ce déplacement, le support continu d'accessoires 12 passe de sa position totalement rétractée dans la lumière 24 à une première position déployée, dans laquelle le premier accessoire 34 situé à l'extrémité avant du chapelet 32 est disposé dans le chemin de guidage 30 en appui sur la tête 20.

L'utilisateur peut alors tremper l'accessoire 34 dans le réservoir 16 de produit cosmétique, puis appliquer du produit cosmétique sur des fibres kératiniques, comme par exemple sur ses cils. En variante, l'utilisateur dépose une dose de produit cosmétique sur l'accessoire 34.

Lorsque l'utilisateur a terminé l'application de produit cosmétique, il déplace encore le bouton de commande 38 dans la fente 28 vers la tête d'appui 20. Comme illustré par l'étape (b) de la Figure 4, le premier accessoire 34A situé à l'extrémité avant du support continu 12 fait alors saillie vers l'avant au-delà de la tête d'appui 20. Un deuxième accessoire 34B adjacent à l'accessoire 34A et situé à l'arrière de celui-ci, est alors disposé dans le chemin de guidage 30 en appui sur la tête d'appui 20.

Le premier accessoire 34A, ainsi que la tige 60 raccordant le premier accessoire 34A au deuxième accessoire 34B font alors saillie au-delà de la tête 20.

L'utilisateur saisit alors la tige 60 avec ses doigts et la fait pivoter vers le bas ou/et la tord autour de son axe pour rompre la liaison frangible 36 au niveau du point de liaison 62.

Comme illustré par l'étape (d) sur la Figure 4, le premier accessoire 34A est alors détaché du reste du chapelet 32. Comme on l'a vu plus haut, la longueur du chapelet 32 et par suite, la longueur L1 du support continu d'accessoires 12, après détachement du premier accessoire 34A sont alors inférieures respectivement à la longueur du chapelet 32 et à la longueur L0 du support continu 12, avant la rupture de la liaison frangible 36 entre le premier accessoire 34A et le deuxième accessoire 34B.

L'utilisateur peut alors utiliser le deuxième accessoire 34B pour l'application du même produit cosmétique ou d'un autre produit cosmétique.

Comme illustré par la Figure 5, les opérations précédentes sont répétées pour utiliser successivement les N accessoires du support en faisant diminuer progressivement la longueur L0 à L4 du support continu et du chapelet 32 à chaque détachement d'un accessoire 34.

Le support continu d'accessoires 12 pouvant être réalisé d'un seul tenant avec les accessoires 34, les liaisons frangibles 36 et le bouton de commande 38 venus de matière, son coût diminue sensiblement. En outre, le distributeur 14 comprenant un corps de préhension 18 pouvant être utilisé successivement avec plusieurs accessoires 34, voire même avec plusieurs supports continus 12 d'accessoires, le dispositif 10 est peu encombrant et simple d'utilisation.

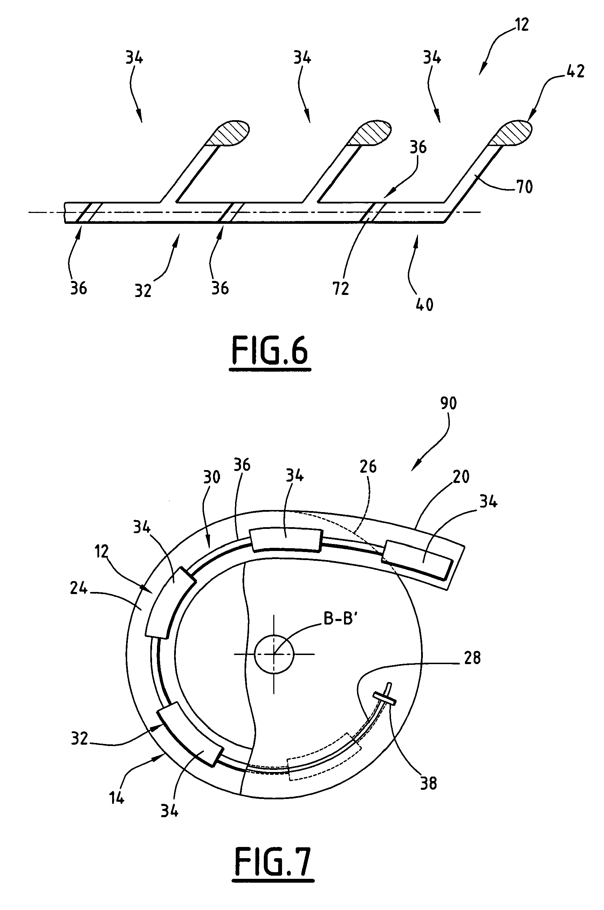

La Figure 6 illustre le support continu 12 d'accessoires d'un deuxième dispositif selon l'invention.

A la différence du premier dispositif 10, le support continu 12 comprend, pour chaque accessoire 34, une base 40 longitudinale dépourvue de moyens d'application de produit cosmétique, et un bras de liaison 70 qui fait saillie transversalement par rapport à la base 40.

L'élément d'application 42 de produit cosmétique destiné à entrer en contact avec les fibres kératiniques de l'utilisateur est disposé à l'extrémité libre du bras 70, à l'écart de la base 40. La base 40 n'est pas destinée à entrer en contact avec les fibres kératiniques de l'utilisateur.

Le bras 70 s'étend suivant un axe B-B' incliné qui forme avec l'axe A-A' un angle non nul compris par exemple entre 90°et 160°.

En outre, les liaisons frangibles 36 sont formées par des lames amincies 72 présentant une section transversale, prise dans un plan sensiblement perpendiculaire à l'axe A-A' inférieure à la section transversale de la base 40.

Le fonctionnement du deuxième dispositif selon l'invention est par ailleurs analogue à celui du premier dispositif 10.

Un troisième dispositif 90 selon l'invention est représenté schématiquement sur la Figure 7. A la différence du premier dispositif 10, le distributeur 14 du troisième dispositif 90 présente une section circulaire, prise dans un plan horizontal, et une forme cylindrique d'axe C-C' perpendiculaire au chemin de guidage 30.

Le chemin de guidage 30 s'étend dans un conduit de forme circulaire ou hélicoïdale d'axe C-C'. Le conduit est situé à la périphérie du distributeur 14.

La tête d'appui 20 fait saillie radialement à l'écart de l'axe B-B'.

Dans ce troisième dispositif 90, le support continu 12 d'accessoires présente une forme circulaire ou hélicoïdale lorsqu'il est disposé dans le distributeur 14.

Le fonctionnement du troisième dispositif 90 selon l'invention est par ailleurs analogue à celui du premier dispositif 10.

Dans une autre variante de dispositif selon l'invention, le distributeur 14 est dépourvu de tête d'appui 20, chaque accessoire 34 étant maintenu en saillie à l'extérieur du distributeur 14 par la liaison frangible 36 lors de l'application du produit cosmétique.

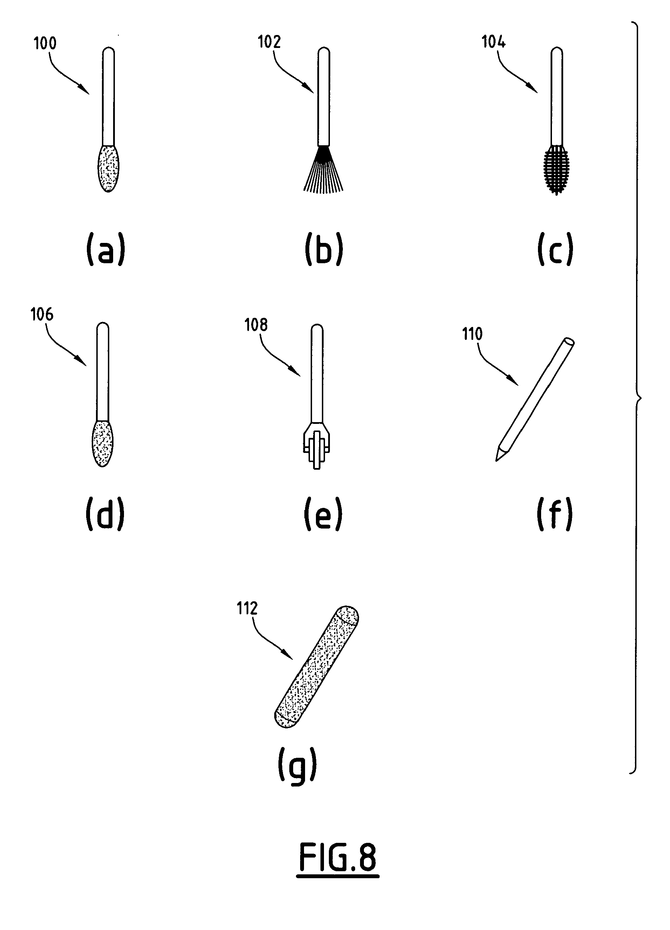

La Figure 8 illustre des variantes d'accessoires cosmétiques 34 destinés à former un chapelet 32 pour des dispositifs selon l'invention.

Les accessoires 34 forment ou comprennent par exemple des applicateurs de produit cosmétique, pourvus d'une mousse, d'un feutre, d'un fritté, d'un tissé, d'un non tissé, ou d'une éponge 100 (variante (a) sur la Figure 8) d'un pinceau 102 (variante (b) sur la Figure 8), d'une brosse à âme torsadée ou injectée ou d'un peigne 104 (variante (c) sur la Figure 8), d'un thermoplastique poreux 106 (variante (d) sur la Figure 8), d'un embout floqué, d'une mousse synthétique habillant une partie moulée ou d'une spatule.

De tels accessoires 34 peuvent également former ou comprendre des outils de traitement cosmétique tel qu'une râpe, une roulette de massage 108 (variante (e) sur la Figure 8), un repousse cuticules 110 (variante (f) sur la Figure 8) ou encore une lime à ongles 112 (variante (g) sur la Figure 8).

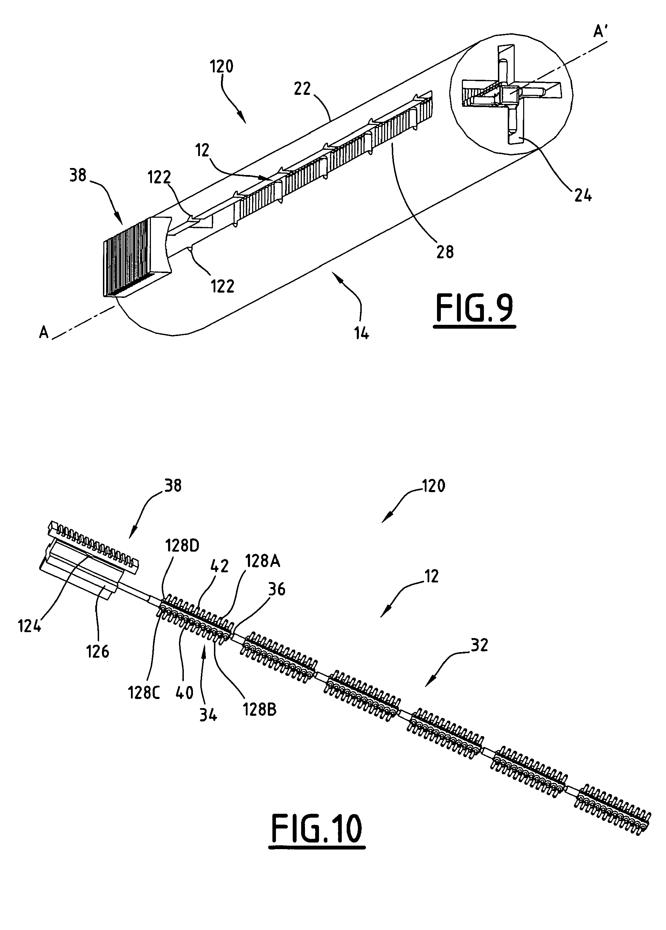

Un quatrième dispositif 120 selon l'invention est représenté sur les Figures 9 et 10.

A la différence du premier dispositif de support 10, la paroi périphérique 22 présente, le long de la fente 28, des encoches transversales 122 en regard, pour l'indexation du déplacement du support continu 12 dans la lumière 24. Le bouton 38 est muni, dans sa partie intérieure engagée dans la fente 28, d'un grain de riz 124 propre à s'engager latéralement dans les encoches 122 pour marquer l'indexation au toucher.

En outre, la lumière 24 présente une section transversale en forme de croix.

Comme illustré par la Figure 10, le bouton 38 comprend un organe de guidage 126 de section transversale complémentaire à celle de la lumière 24 pour guider le déplacement du support d'accessoires 12 dans la lumière 24 le long de l'axe A-A'.

Le chapelet 32 d'accessoires 34 comprend une pluralité d'accessoires 34 reliés entre eux par des liaisons frangibles 36, chaque accessoire 34 présentant des éléments d'application 42 disposés en forme de croix autour de la base centrale 40 d'axe A-A'.

Ainsi, chaque accessoire 34 comprend deux rangées 128A, 128B longitudinales d'éléments d'application 42 formant un peigne. Les rangées 128A, 128B font saillie transversalement dans un premier plan axial médian, dans des sens opposés à partir de la base 40.

Chaque accessoire 34 comprend en outre deux rangées longitudinales perpendiculaires 128C, 128D d'éléments d'application 42 formant un peigne. Ces rangées perpendiculaires 128C, 128D s'étendent dans un deuxième plan axial médian perpendiculaire au premier plan axial en faisant saillie à partir de la base 40, à l'opposé l'une de l'autre.

Ainsi, les accessoires 34 sont reçus de manière sensiblement complémentaire dans la fente 24 en forme de croix, avec chaque rangée 128A, 128B, 128C, 128D reçue dans une extension latérale distincte de la croix.

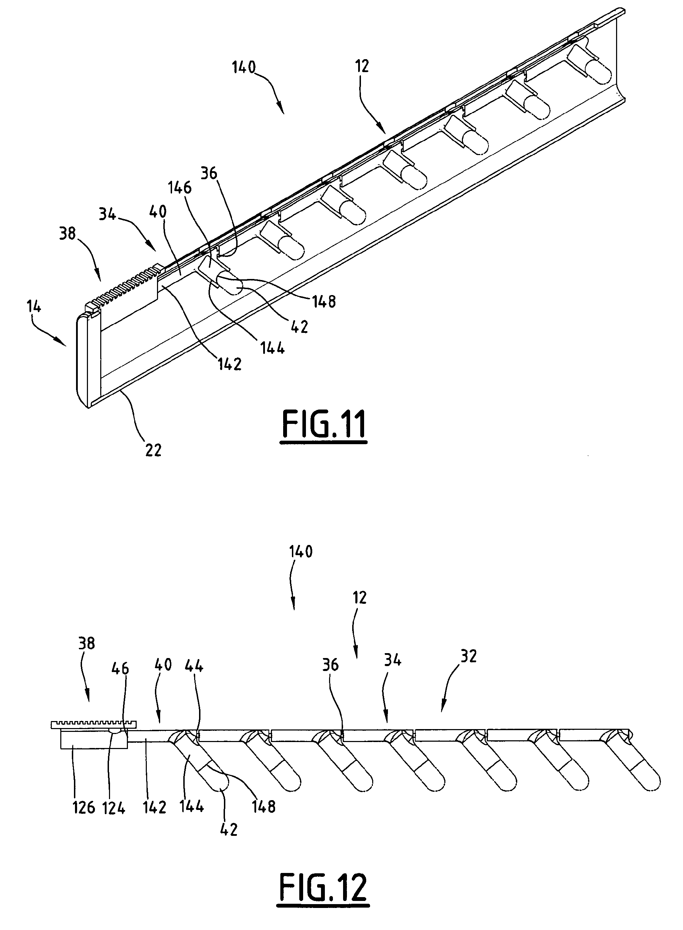

Un cinquième dispositif 140 selon l'invention est représenté sur les Figures 11 et 12. A la différence du premier dispositif 10, la paroi périphérique 22 est de forme sensiblement ovale généralement allongée dans un plan médian passant par la fente 28, ce plan étant vertical sur la Figure 11.

A la différence du premier dispositif 10, la base 40 de chaque accessoire 34 du chapelet 32 comprend une membrure axiale 142 et une cupule 144 de réception d'un élément d'application 42. L'élément 42 est représenté schématiquement en traits semi-pleins sur la Figure 11 et sur la Figure 12.

La membrure 142 est pleine et elle s'étend entre la paroi avant 44 et la paroi arrière 46.

La cupule 144 fait saillie transversalement par rapport à la membrure 142. Elle délimite un logement interne 146 de réception de l'élément d'application 42.

Dans cet exemple, la cupule 144 est de forme sensiblement cylindrique et s'étend suivant un axe incliné par rapport à l'axe A-A' d'un angle supérieur à 10° et sensiblement égal à 45°dans l'exemple de la Fig ure 12.

Le logement 146 est obturé vers l'axe A-A' par la membrure 42 et débouche à l'écart de l'axe A-A' par une ouverture 148 d'insertion de l'élément d'application 42. L'élément d'application 42 est ainsi monté dans la cupule 144 en présentant un applicateur qui fait saillie à l'écart de l'ouverture 148. Cet applicateur est par exemple un embout floqué, une éponge, un pinceau ou l'un quelconque des applicateurs décrits sur la Figure 8 ou dans ce qui précède.

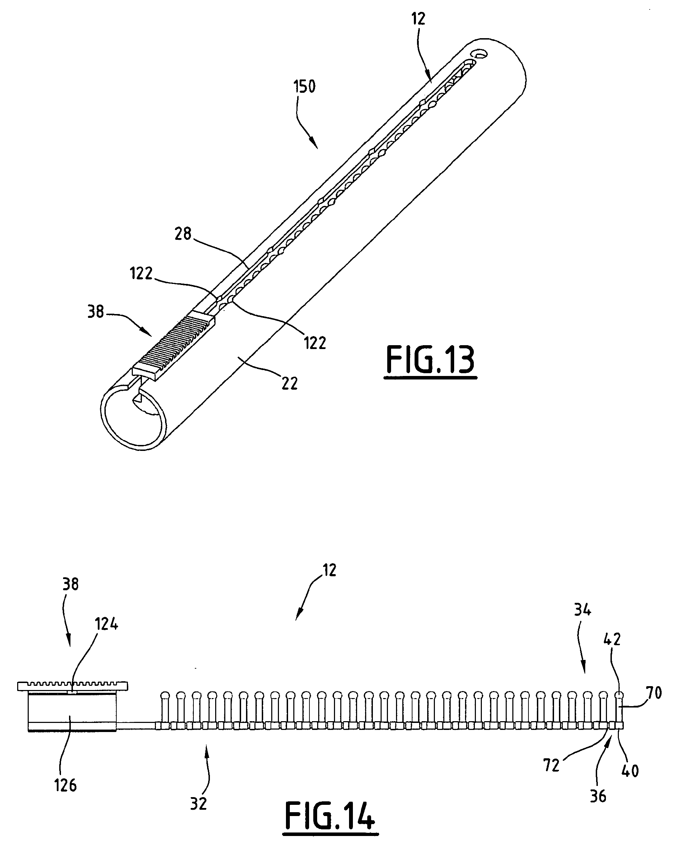

Les Figures 13 et 14 illustrent un sixième dispositif 150 selon l'invention.

A la différence du deuxième dispositif selon l'invention représenté sur la Figure 6, l'élément d'application 42 comprend à son extrémité libre une tête de produit sec chargé sur l'applicateur.

Ce produit sec est destiné à être mélangé avec un produit fluide avant l'application du mélange sur l'utilisateur.

Lors de l'utilisation du sixième dispositif 150, l'élément d'application 42 comprenant le produit sec, est trempé dans le produit fluide avant application du mélange sur une surface corporelle d'un utilisateur.

En outre, les bras 70 sont sensiblement perpendiculaires à l'axe A-A' dans l'exemple représenté sur les Figures 13 et 14.

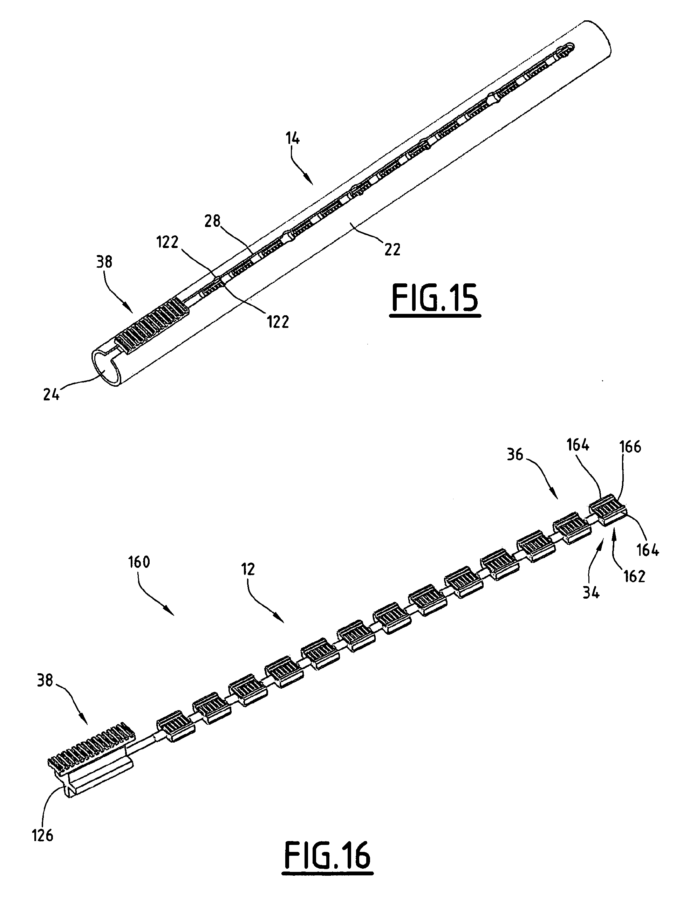

Un septième dispositif 160 selon l'invention est représenté sur les Figures 15 et 16. A la différence du premier dispositif 10, la base 40 de chaque accessoire 34 délimite un réceptacle 162 propre à recevoir un bloc de produit cosmétique. Le réceptacle comprend deux rebords latéraux 164 et un fond muni de stries transversales 166 pour accrocher le bloc de produit cosmétique destiné à être chargé dans le réceptacle 162.

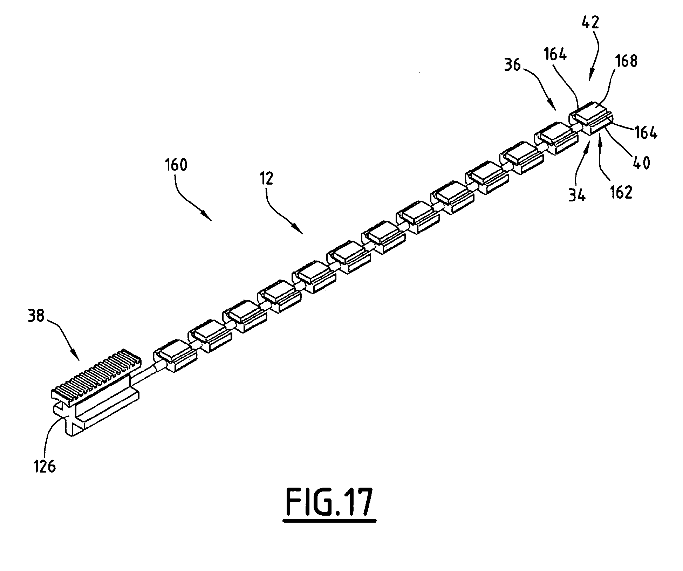

Comme illustré par la Figure 17, chaque réceptacle 162 est chargé, lors de la fabrication du septième dispositif 160, ou préalablement à l'utilisation, par un bloc 168 de produit cosmétique qui est fixé dans le réceptacle 162 entre les rebords 164. La fixation est par exemple effectuée par encliquetage dans les stries 166.

Le fonctionnement du septième dispositif 160 est par ailleurs analogue à celui du premier dispositif 10.

## Revendications

1. Dispositif (10 ; 90 ; 120 ; 140 ; 150 ; 160) de distribution d'accessoires cosmétiques (34), du type comprenant :
- au moins un support (12) d'accessoires comprenant une pluralité d'accessoires cosmétiques (34), chaque accessoire cosmétique (34) étant détachable par rapport aux autres accessoires cosmétiques (34) du support (12) d'accessoires;
- un distributeur (14) d'accessoires cosmétiques délimitant un chemin de guidage (30) du support (12) d'accessoires, le support (12) d'accessoires étant déplaçable suivant sa longueur dans le chemin de guidage (30) entre une position rétractée dans le distributeur (14) et une pluralité de positions déployées d'utilisation d'un accessoire (34) ;
**caractérisé en ce que** le support (12) d'accessoires comprend au moins un chapelet (32) d'accessoires cosmétiques (34) disposés bout à bout en étant reliés entre eux par des liaisons frangibles (36), la rupture d'une des liaisons frangibles (36) pour détacher un accessoire cosmétique (34) du support (32) provoquant une diminution de la longueur du chapelet (32) d'accessoires, prise le long du chemin de guidage (30).

2. Dispositif (10 ; 90 ; 120 ; 140 ; 150 ; 160) selon la revendication 1, **caractérisé en ce que** la pluralité d'accessoires cosmétiques (34) et les liaisons frangibles (36) du chapelet d'accessoires (32) sont venues de matière.

3. Dispositif (10 ; 90; 120 ; 140 ; 150 ; 160) selon l'une quelconque des revendications 1 ou 2, **caractérisé en ce** chaque accessoire cosmétique (34) comprend :
- une base longitudinale (40) raccordée à une base longitudinale (40) d'au moins un accessoire adjacent (34) par une liaison frangible (36) ; et
- au moins une partie transversale (54 ; 70) qui fait saillie transversalement par rapport à la base longitudinale (40).

4. Dispositif (10 ; 90 ; 120 ; 160) selon la revendication 3, **caractérisé en ce que** la base (40) et la partie transversale (54) forment un embout applicateur de produit cosmétique.

5. Dispositif (10 ; 90 ; 140) selon la revendication 3, **caractérisé en ce que** la partie transversale (70) comprend un bras de liaison (70) qui fait saillie transversalement par rapport à la base longitudinale (40), et un embout applicateur (42) de produit cosmétique disposé à une extrémité libre du bras (70).

6. Dispositif (10 ; 90 ; 120 ; 140 ; 150 ; 160) selon l'une quelconque des revendications précédentes, **caractérisé en ce que** chaque liaison frangible (36) comprend un organe longitudinal (60) présentant un premier point de liaison (62) avec un premier accessoire (34) du chapelet d'accessoires (32) et un deuxième point de liaison (64) avec un deuxième accessoire (34) du chapelet d'accessoires (32).

7. Dispositif (10 ; 90) selon la revendication 6, **caractérisé en ce qu'**au moins le premier accessoire (34) présente un bord (52) de protection d'utilisateur qui fait saillie longitudinalement par rapport au premier point de liaison (62) vers le deuxième accessoire (34), le bord de protection (52) s'étendant longitudinalement en regard de l'organe longitudinal (60).

8. Dispositif (10 ; 120 ; 140 ; 150 ; 160) selon l'une quelconque des revendications précédentes, **caractérisé en ce que** le chemin de guidage (30) défini par le distributeur (14) est linéaire, le support (12) d'accessoires s'étendant linéairement au moins lorsqu'il est disposé dans le chemin de guidage (30) du distributeur (14).

9. Dispositif (90) selon l'une quelconque des revendications 1 à 7, **caractérisé en ce que** le chemin de guidage (30) défini par le distributeur (14) est curviligne, le support (12) d'accessoires adoptant une configuration curviligne au moins lorsqu'il est disposé dans le chemin de guidage (30) du distributeur (14).

10. Dispositif (10 ; 90; 120 ; 140 ; 150 ; 160) selon l'une quelconque des revendications précédentes, **caractérisé en ce que** le support (12) d'accessoires comprend un organe (38) de déplacement du chapelet d'accessoires (32) dans le distributeur (14), monté solidaire et distinct du chapelet d'accessoires (32), l'organe de déplacement (38) étant propre à être saisi par un utilisateur du dispositif (10 ; 90) à travers le distributeur (14) pour déplacer le chapelet d'accessoires (32) dans le distributeur (14) le long du chemin de guidage (30).

11. Dispositif (10 ; 90) selon l'une quelconque des revendications précédentes, **caractérisé en ce que** le distributeur (14) comprend :
- un corps creux (18) de réception du support (12) d'accessoires ; et
- une tête d'appui (20) faisant saillie à partir du corps creux (18), le chemin de guidage (30) s'étendant à travers le corps creux (18) et à travers la tête d'appui (20), chaque accessoire (34) étant recouvert par le corps creux (18) pour empêcher l'utilisation de l'accessoire (34) lorsqu'il est disposé dans le corps creux (18), l'espace situé au-dessus d'un accessoire (34) étant dégagé pour autoriser l'utilisation de l'accessoire (34), lorsqu'il est disposé dans la tête d'appui (20).

12. Dispositif (10 ; 90; 120 ; 140 ; 150 ; 160) selon l'une quelconque des revendications précédentes, **caractérisé en ce que** le chemin de guidage (30) du distributeur (14) est délimité par une rainure ou une gorge de section transversale sensiblement conjuguée à la section transversale d'au moins un accessoire cosmétique (34) du support (12) d'accessoires.

13. Dispositif (10 ; 90; 120 ; 140 ; 150 ; 160) selon l'une quelconque des revendications précédentes, **caractérisé en ce que** chaque accessoire cosmétique (34) est choisi parmi un applicateur de produit cosmétique ou un outil de traitement cosmétique.

14. Procédé de préparation d'un dispositif (10 ; 90; 120 ; 140 ; 150 ; 160) selon l'une quelconque des revendications précédentes en vue de l'utilisation d'un accessoire cosmétique (34), **caractérisé en ce qu'**il comprend les étapes suivantes :
- déplacement du support (12) d'accessoires dans le chemin de guidage (30) jusqu'à une position déployée ;
- rupture d'une liaison frangible (36) entre un premier accessoire cosmétique (34A) et un deuxième accessoire cosmétique (34B), la rupture de la liaison frangible (36) et le détachement du premier accessoire cosmétique (34B) provoquant une diminution de la longueur du chapelet d'accessoires (32), prise le long du chemin de guidage (30).
